# EUROPEAN PATENT SPECIFICATION

(11) **EP 2 724 724 B1**
(45) Date of publication and mention of the grant of the patent: **28.09.2016**
(21) Application number: 13190202.5
(22) Date of filing: 25.10.2013
(51) Int. Cl.: A61K 33/30, A61K 33/34, A61P 17/00

(54) **Treatment and prevention of epithelial infections with copper and zinc chelates**
Behandlung und Prävention von Epithelinfektionen mit Zink und Kupfer chelaten
Traitement et prévention des infections épithéliales par des chélates de zinc et de cuivre

(30) Priority: 26.10.2012 NL 2009707
(43) Date of publication of application: 30.04.2014
(73) Proprietor: Intracare B.V., 5466 AZ Veghel (NL)
(72) Inventor: Vulders, Carly, 5466 AZ Veghel (NL)
(74) Representative: Paemen, Liesbet R.J.

(56) References cited:
- WO-A1-02/060433
- A. RELUN ET AL: "Effectiveness of different regimens of a collective topical treatment using a solution of copper and zinc chelates in the cure of digital dermatitis in dairy farms under field conditions", JOURNAL OF DAIRY SCIENCE, vol. 95, no. 7, July 2012 (2012-07), pages 3722-3735, XP055095261, ISSN: 0022-0302, DOI: 10.3168/jds.2011-4983
- M. HOLZHAUER ET AL: "Curative effect of topical treatment of digital dermatitis with a gel containing activated copper and zinc chelate", VETERINARY RECORD, vol. 169, no. 21, 27 September 2011 (2011-09-27), pages 555-555, XP055095286, ISSN: 0042-4900, DOI: 10.1136/vr.d5513

## Description

### FIELD OF THE INVENTION

The present inventions relates to sprayable metal chelate formulations for the treatment and prevention of bacterial infections of epithelia.

### BACKGROUND OF THE INVENTION

Farm animals such as swine, sheep, goats, horses, and cattle are prone to infectious diseases of the skin, such as exudative epidermitis, digital dermatitis (hairy hoof warts), hoof rot (interdigital phlegmon), stable hoof rot (interdigital dermatitis), and udder cleft dermatitis.

These conditions cause stress to the animals and can affect weight gain, reproductive rates, and in the case of sheep, wool production.

Skin disease in a swine herd can cause a significant decrease in growth rate and feed efficiency. Hoof ailments in milk cattle may result in reduced milk production, reduced fertility, and increased risk of mastitis. Furthermore, skin ailments demand much more work and more treatment costs often leading to premature removal of the affected animals.

Exudative epidermitis is caused by Staphylococcus hyicus and is most commonly observed in piglets of 1-8 weeks of age. Lesions can progress from the face to the rest of the body and become strongly exudating. The only known treatment is with antibiotics.

Foot rot or hoof rot is a bacterial infection of the soft tissue between the two toes of the animal. It is very painful and contagious quickly spreading and affecting the whole herd. The bacteria causative of foot rot are common to the environment in which the animals live.

Usually, there is an injury to the skin between the hooves that allows the bacteria to infect the animal. Foot rot may also be promoted by high temperatures and humidity, causing the skin between the hooves to crack and let the bacteria infect the foot. The first signs of hoof rot are limping, holding limbs above the ground, grazing on knees, and reluctance to walk.

Animals with foot rot might show interdigital inflammation, fever, loss of appetite and accompanying weight loss, and develop mild to severe lameness with hoof deformity.

Animals with chronic infections show a loss of body condition and decreased production, resulting in an unhealthy animal overall.

Udder cleft dermatitis is skin disease commonly observed in dairy herds, particularly in heifers. However, the disease can be present at all stages of lactation, and even during non-lactating periods. Characteristic features of udder cleft dermatitis are a moist appearance and necrosis of the skin, and a foul odor. Lesions are mostly located bat the cranial edge of the cleft between the two cranial quarters, although it can also be centered between all four quarters.

Digital Dermatitis, also known as "Papillomatous digital dermatitis" (PDD) or "Mortellaro's Disease", "strawberries", "hairy foot warts", "Interdigital Papillomatous", "strawberry Heel", or "Italian foot rot" is an infectious claw disease attributed to bacterial infection. In the last years it has become more and more common in livestock farming. This disease can be recognized by an inflammation of the skin in the area where skin changes into claw horn. The inflammable spot is reddish and looks a bit like a strawberry. Most often the hairs are standing upright around the affected areas

Interdigital dermatitis (not be confused with hoof rot), or stable hoof rot, is a chronic inflammation of the skin in the area between the toes of the feet (interdigital cleft). This infection is caused by the bacterium Treponema. The skin in the area of the interdigital cleft appears puffy with a dry exudation causing a crust to form.

The infected animals are preferably separated away from the herd as soon as possible to prevent the infection from spreading. Treatment consists in cleaning the foot thoroughly, clipping the hoof with subsequent treatment with an antimicrobial product. Infectious diseases of the hoof can be prevented and treated by utilizing a foot bath or footwrap with zinc sulfate and copper sulfate, but also other germicides such as hydrogen peroxide, or even an antibiotic can be used.

WO 02/060433 describes aqueous compositions comprising copper and zinc chelates for use in the prevention and treatment of inflammations of the skin and the hoofs of animals. It is generally mentioned that such compositions may be applied in the form of a spray, which implies that in these aqueous compositions the copper and zinc chelates are dissolved.

WO 98/20749 describes aqueous nutritive preparations comprising lactic acid (or derivatives thereof) and chelates of trace elements such as copper and zinc. The composition may be used for treating hoofs of animals and may be applied directly to the hoof.

A product containing copper diammonium EDTA and zinc diammonium EDTA is sold under the trademark Intra Hoof-Fit Gel marketed by Intracare and is registered as Dutch registration REG NL 109138. The Intra Hoof-Fit Gel product more particularly copper and zinc diammonium ethylenediaminetetraacetic acid (EDTA) and may be used for the treatment or prevention of hoof infections such as Mortellaro, and is typically applied to the hoof via a brush.

The above formulations are all fluids which are incorporated into a gel.

The use of copper salts in the treatment of hoof infectious diseases presents environmental concerns. For example after usage in a foot bath, the remaining solution is typically drained into the manure store and is subsequently applied to the fields. Also other types of application of copper salts to the animal's hoofs result in the release of copper compounds in the environment. Excess copper can be hazardous to the cattle and sheep. The application of the copper-laden manure onto the fields causes a copper build-up, which can be problematic to the plants' development and, therefore, should be avoided.

Therefore, a need exists for a treatment that is effective against hoof infections, in particular foot rot, stable foot rot, and PDD, that is affordable, that is convenient to apply and that is environmentally-friendly. One or more of these needs are met by the treatment of the present invention.

### SUMMARY OF THE INVENTION

Provided herein are formulations, more particularly sprayable formulations, comprising micronized copper and zinc chelates for use in the treatment and prevention of bacterial infections of epithelial cells, more particularly of animals, wherein said copper and zinc chelates are micronized and suspended in a liquid comprising less than 5 % (w/v) water, such that the total amount of copper and zinc chelate in said formulation ranges from 5 to 50 percent by weight; and less than 5% (w/v) of said copper and zinc chelates are dissolved in said liquid.

Indeed, it has been found that the envisaged compositions provide an effective alternative to the use of antibiotics, which have a number of disadvantages (potential contamination of milk and meat, resistance). Moreover, in particular embodiments, the treatment methods described herein are more effective and/or more efficient (in that they require application of a more limited volume) than existing treatment options with copper and zinc compounds.

This allows the administration of reduced dosages of copper and zinc and a concomitant reduction of side effects. The methods described herein are moreover more environment-friendly in that less of the metals of the formulation enter into the environment, thereby avoiding the side effects related to the exposure of the environment to copper and zinc compounds. Finally, the administration thereof can be ensured in a more hygienic way, avoiding cross-contamination.

Thus the compositions envisaged herein may involve one or more advantages such as introduction of less metal in the environment, increased effectiveness, longer duration of action, fewer administrations, etc. The formulations of the invention in addition allow a more focused administration of the active ingredients, with limited spoilage, in that they can be sprayed directly on the affected region, in particular directly on the infected epithelial cells such as but not limited to the skin, or hooves of the animal. In particular embodiments, the methods describes direct administration on the tissue between the toes. The formulations offer the additional advantage of showing prolonged activity so that less frequent administrations are required. They moreover allow need-tailored administration in function of the severity of the symptoms.

In particular embodiments, the treatment and prevention of bacterial skin infections is described, i.e. treatment and prevention of infections of dermis and epidermis is envisaged. In particular embodiments, the conditions envisaged are related to the infection of the hoof of animals. In certain embodiments, the conditions envisaged are related to the infection of the udder of animals, more particularly the udder of dairy cattle.

In particular embodiments, the application thus provides compositions for use in the treatment and prevention of bacterial infections and conditions related to bacterial infections of epithelial cells in an animal wherein said infections are selected from the list consisting of infections of any epithelial cell, of the skin and the hooves of animals, selected from the list consisting of: greasy pig disease (exudative epidermitis), porcine cutaneous spirochetosis, porcine necrotic ear syndrome, hairy hoof warts or Mortellaro's Disease (digital dermatitis), hoof rot (interdigital phlegmon), stable hoof rot (interdigital dermatitis), udder cleft dermatitis and M1 (early stage) and/or M2 (ulcerative stage) lesions on the skin or in the hoof and conditions selected from irritation (including reddening, swelling and other phenomena), pain and inflammation.

In particular embodiments of the composition for use as provided herein, the micronized copper and zinc chelates are suspended in a non-aqueous liquid.

In particular embodiments of the composition for use as provided herein, the liquid is selected from propanol, isopropanol, butanol, isobutanol, or a mixture thereof. More particularly, the liquid is propanol.

In particular embodiments of the composition for use as provided herein, the copper chelate : zinc chelate molar ratio of said copper and zinc chelates ranges from 2 : 1 to 1 : 2.

In particular embodiments of the composition for use as provided herein, the composition further comprises a silicate.

In particular embodiments of the composition for use as provided herein, less than 2% (w/v) of said copper and zinc chelates are dissolved in said liquid.

In particular embodiments of the composition for use as provided herein, the copper and zinc chelates are provided as microparticles, having a mean surface weighted diameter ranging from 0.5µm to 10µm.

In a further aspect, sprayable formulations comprising micronized copper and zinc chelatesare provided comprising less than 5 % (w/v) water, wherein the total amount of copper and zinc chelate in said formulation ranges from 5 to 50 percent by weight; and less than 5% (w/v) of said copper and zinc chelates are dissolved in said liquid. In particular embodiments, sprayable compositions are provided comprising micronized copper and zinc chelates suspended in a liquid, more particularly a skin-tolerable non-aqueous liquid.

In particular embodiments, the copper and zinc chelates are EDTA chelates of copper and zinc, in particular disodium of copper(EDTA) and of Zn(EDTA). In particular embodiments, the liquid is selected from propanol, isopropanol, butanol, isobutanol, or a mixture thereof.

A further aspect relates to the spray can comprising a gas propellant and a composition according to the invention.

### DETAILED DESCRIPTION OF THE INVENTION

The term "about", when used in relation to a numerical value, has the meaning generally understood in the relevant art. In certain embodiments the term "about" may be left out or may be interpreted to mean the numerical value +10%; or +5%; or +2%; or +1 %.

Whenever used herein in relation to a percentage, w/w means weight/weight and w/v means weight/volume.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.

Furthermore, the terms first, second, third and the like in the description and in the claims, are used for distinguishing between similar elements or steps and not necessarily for describing a sequential or chronological order, unless specified. It is to be understood that the terms so used are interchangeable under appropriate circumstances and that the embodiments described herein are capable of operation in other sequences than described or illustrated herein.

Reference throughout this specification to "one embodiment" or "an embodiment" means that a particular feature, structure or characteristic described in connection with the embodiment is included in at least one embodiment envisaged herein. Thus, appearances of the phrases "in one embodiment" or "in an embodiment" in various places throughout this specification are not necessarily all referring to the same embodiment, but may.

Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The present application relates to compositions for the treatment of bacterial infection of epithelial cells of animals, and conditions associated with or resulting from such infections.

The term "infection of epithelial cells of animals", as used herein, refers to infections of any epithelial cell, but more particularly the skin and the hooves of animals. These infections include but are not limited to the infections described above, in particular greasy pig disease (exudative epidermitis), porcine cutaneous spirochetosis, porcine necrotic ear syndrome, hairy hoof warts (digital dermatitis), hoof rot (interdigital phlegmon), stable hoof rot (interdigital dermatitis), and udder cleft dermatitis. The term is also meant to cover conditions associated with or resulting from epithelial cell infections, in particular irritation (including reddening, swelling and other phenomena), pain, inflammation, more in particular skin inflammation. In particular embodiments, the "conditions related to epithelial cell infections" include reduced growth, reduced reproductive rates, and in the case of sheep, reduced wool production, less milk production, increased risk of mastitis, as well as those conditions further mentioned herein. The conditions may be acute as well as chronic.

The compositions envisaged herein are in particular suitable for use in the treatment and prevention of bacterial dermatoses, such as Greasy pig disease, porcine necrotic ear syndrome, porcine cutaneous spirochetosis, Mortellaro's Disease (digital dermatitis), hoof rot, including inflammatory conditions associated therewith. It was further found that the compositions described herein are surprisingly effective for use in the treatment and prevention of udder cleft dermatitis, including inflammatory conditions associated therewith.

In particular embodiments, the compositions envisaged herein are used for the treatment of M1 (early stage) and/or M2 (ulcerative stage) lesions on the skin or in the hoof.

Although the compositions envisaged herein may be applied on any (ungulate) animal, the application on farm animals such as sheep, goats, horses, pigs and cattle is particularly envisaged.

In particular embodiments, the application method described herein is by spraying the composition on the skin, more particularly the area of infection or susceptible of infection. In further particular embodiment, the area of infection is characterized by signs of inflammation.

In further particular embodiments, the compositions are sprayed directly onto lesions present on the skin.

This implies that the compositions envisaged herein are typically provided as sprayable compositions, meaning that they can be administered using a suitable spraying device. For example the viscosity will be sufficiently low, e.g. below 10 mPa.s. Accordingly, provided herein are sprays for liquid compositions comprising the compositions envisaged herein.

The compositions for use as claimed herein are characterized in that they comprise copper and zinc chelates wherein said copper and zinc chelates are micronized and suspended in a liquid comprising less than 5 % (w/v) water, such that the total amount of copper and zinc chelate in said formulation ranges from 5 to 50 percent by weight; and less than 5% (w/v) of said copper and zinc chelates are dissolved in said liquid. In any given case, the quantity of the formulations of zinc and copper chelate administered to the animal to be treated, and therefore also the quantity of the zinc and copper chelate active ingredients therein, will be adjusted in accordance with the purpose to be attained (prevention or therapy), the nature of the subject, the condition under consideration as well as the severity of the condition, the type of the formulation concerned, and any other relevant facts that may modify the activity of the zinc and copper chelate or the response of the subject, as is well known by those skilled in the art.

The formulations of the invention may be administered once daily or multiple times daily, for example two, three, or four times per day. In particular embodiments, at least two administrations are envisaged within a time period of one week. In further particular embodiments, two administrations are envisaged separated by 2 days (i.e. day 0 and day 3). In further particular embodiments the methods described involve spraying the infected hoof. In certain embodiments, one or more daily administrations are envisaged per day, during a period of at least one week, at least 10 days, or at least two weeks.

In particular embodiments, the methods described herein attain a reduction of inflammation within 10 days after the first treatment.

In particular embodiments, the copper and zinc chelates are EDTA chelates of copper and zinc, in particular the salts of EDTA chelates of copper and zinc. Of interest are the dialkalimetal salts of copper[EDTA]²- and of Zn[EDTA]²⁻. Of further interest are the disodium salts of copper(EDTA) and of Zn(EDTA) complex. These salts may also be referred to as copper and zinc disodium ethylenediaminetetraacetic acid or ethylenediaminetetraacetate-copper- and zinc-sodium complex.

The disodium salts of copper[EDTA]²⁻ and of Zn[EDTA]²⁻ in particular are attractive due to their effectiveness, thereby reducing amount of copper and zinc to be administered, and reducing concomitant side effects.

In the formulations for use envisaged herein, the copper and zinc chelates are suspended in a liquid. In particular embodiments, the fluid serves merely as a carrier fluid and evaporates easily leaving behind the particles on the area of interest. In particular embodiments the chelates may be suspended in the form of micronized particles in a skin-tolerable liquid e.g. non-toxic, causing no irritation or allergic reactions. In particular embodiments the liquids are evaporative (volatile) and skin-compatible. The term "evaporative liquid" or "volatile liquid" as used herein refers to a liquid having a vapor pressure higher than the vapor pressure of water at a temperature of about 25°C.

The liquid in which the micronized particles are suspended is selected such that substantially none of the copper or zinc chelate dissolves, the term substantially none meaning that no or a small amount of the copper and zinc chelates present in the formulation dissolves, i.e. less than 5%(w/v). Said liquid is non-aqueous, although minor amounts of water, i.e. less than 5 %(w/v) can be present.

The formulations envisaged herein typically contain at least 45 w% of evaporative liquids, preferably at least 50 w%, and more preferably at least 60 w%, based on the total weight of the formulation excluding optional propellants.

Exemplary evaporative liquids that can be used include alcohols, in particular alkanols such a propanol, isopropanol, butanol, isobutanol, including mixtures thereof. They may contain small amounts of water, i.e. such amounts of water that do not cause the micronized particles to dissolve. In particular embodiments, the liquid comprises less than 20% (w/w) water, more preferably less than 10% (w/w) water, most preferably less than 5% (w/w) water.

The total amount of copper and zinc chelate in the present formulations ranges from 5-50 per cent by weight, based on the total weight of the formulation. In further particular embodiments the total amount of copper and zinc chelate ranges from 10-40 per cent by weight. In further particular embodiments the concentration is about 30 per cent by weight. In certain embodiments the total amount of copper and zinc chelate ranges from 10-40 per cent by weight, based on the total weight of the formulation excluding optional propellants. In further embodiments, the total amount of copper and zinc chelate ranges from 20-40 per cent by weight, more particularly about 30 per cent by weight, based on the total weight of the formulation excluding optional propellants.

The copper and zinc chelates may be present in a copper chelate : zinc chelate molar ratio that is in the range of about 2 : 1 to about 1 : 2, or about 1.5 : 1 to about 1 : 1.5, the molar ratio between these chelates preferably being about 1 : 1.

The micronized zinc or copper chelates are present in the formulations envisaged herein as microparticles. In one embodiment, the mean surface weighed diameter is in the range of about 0.5 to about 10 µm, or about 1 to about 5 µm, or about 2 to about 4 µm, or, preferably about 2.2 to about 2.8 µm. Or the mean volume weighed diameter is in the range of about 1 to about 10 µm, or about 2 to about 5 µm, or about 3 to about 5 µm, or about 3.5 to about 4 µm. The size or diameter of the microparticles can be determined as an equivalent spherical diameter via techniques such as Dynamic Light Scattering. Microparticles of the copper and zinc chelates provide the advantage that they can be sprayed. Indeed, providing the chelates as microparticles may allow for obtaining compositions having a high chelate content, which still have a suitable viscosity for spraying. Moreover, the ratio between surface and content is such that the particle will easily dissolve in the wound fluid.

In particular embodiments, the microparticles have a size of about 4µm. The effectiveness of these microparticles results in less of the salts having to be administered with fewer concomitant side effects.

The span of the microparticle distribution may be in the range of about 50 to about 0.1, preferably in the range of about 3 to about 0.1, in particular about 3 to about 1, or about 2 to about 1, and more preferably about 1.5 to about 1.7.

The particles of the chelates are preferably present as a monomodal distribution.

Each of the copper and zinc chelates in micronized form can be prepared separately or they can be prepared in combination. When prepared separately, the micronized powders or micronized dispersions can be mixed subsequently. Also end formulations or intermediate formulations with only copper chelate and zinc chelate can be prepared and subsequently mixed.

The copper and zinc chelates in micronized form can be prepared using techniques known in the art. Such methods include milling, bashing and grinding. The mechanical means applied to reduce the effective average effective particle size can include a ball mill, an attritor/attrition mill, a vibratory mill, a planetary mill, media mills, such as a sand mill and a bead mill.

The micronized copper and zinc chelates can also be prepared by wet milling of the starting copper and zinc chelates as a dispersion in a suitable liquid medium in which they are essentially insoluble, for example in any of the skin-tolerable non-aqueous liquids mentioned herein.

A bead mill can be used with ceramic or metal beads. The milling can be done in a one step or multistep procedure, where in the latter instance different mills and/or beads can be used. Prior to milling, the particle size of the material may be reduced first reduced to a particular size, e.g. a size of less than about 100 µm, which after sieving may be processed further.

The formulations envisaged herein may contain other therapeutically active ingredients such as antibiotics, including antibacterials and antifungals. In particular embodiments however, the compositions do not comprise antibiotics, more particularly they do not comprise specific antibiotics. Indeed, it was found that the compositions described herein are surprisingly effective for the treatment of epithelial infections, without requiring the addition of antibiotics.

In particular embodiments the formulations of the present invention comprise copper and zinc chelates as the only chelates present in the formulation. In further particular embodiments the formulation comprises copper zinc disodium EDTA as the only active ingredient.

The formulations for use in the invention may further contain adjuvants, such as preservatives, stabilizing agents, anti-agglomeration agents, etc. In particular embodiments, the compositions comprise one or more silicates, such as Bentone gel®. In certain embodiments, the compositions comprise between 0.1 w% and 2 w% of silicates.

They may also contain ingredients that improve the spraying characteristics of the formulations or the color. The addition of dyes to the composition may facilitate the identification of the areas on the skin which have been treated with the composition. Suitable dyes for use in the composition include but are not limited to Brilliant blue FCF, Patent blue V, and curcumin. In certain embodiments, the compositions comprise between 0.01 w% and 1 w% of dyes, more particularly between 0.05 w% and 0.5 w%.

The formulations for use in the invention can be administered by various devices, which may be sterile or non-sterile. Such devices comprise multi-dose or unit-dose containers or unit-dose sprays or any other container, including glass bottles with a spray device and spraying cans with or without the use of propellants. In the latter instance the sprayable formulations may contain 5 - 80% w/w, in particular 10 - 80 % w/w of a propellant. In further embodiments, the formulations may contain 10 - 70% w/w, or 20 - 60% w/w, of a propellant.

Propellants can be liquefied gas propellants or compressed gas propellants: Liquified propellants that can be used are hydrocarbons with a boiling point below room temperature such as C₃₋₄ hydrocarbons, in particular propane or butane, dimethylether or chlorofluorocarbons. Alternative propellants include but are not limited to compressed gas propellants such as compressed air and Nitrogen. The propellant produces a pressure in the container such that upon usage it expels the formulation out of the container. In particular embodiments, the propellant is LPG with various components.

Using a spraying device significantly facilitates the application of the copper and zinc chelate formulation on the area of the hoof to be treated.

The following examples are meant to illustrate the present invention and should not be construed as a limitation of its scope.

### EXAMPLES

### A. Composition

An exemplary composition of a sprayable formulation according to a particular embodiment of the present invention comprises copper and zinc cations as active ingredients, in the form of copper disodium EDTA and zinc disodium EDTA, respectively. The composition (including excipients such as propellants) comprises about 5 w% of each chelate.

The formulation further comprises a number of excipients. Isopropyl alcohol is used as a carrier liquid, and colloidal anhydrous silica is used as a rheological additive. Butane and propane are used as propellants. The formulation further comprises a number of dyes: Brilliant blue FCF, Patent blue V, and curcumin. The dyes provide a clear indication of the treated area of the skin.

The formulation was used for the treatment of digital dermatitis and udder cleft dermatitis in a number of field trials, as discussed below.

### B. Treatment of digital dermatitis (DP) - Field trial with qualitative evaluation

Cattle with DD infections: M1 and M2 lesions were evaluated and treated.

On day 0 the claw of the infected animal was clipped and cleaned and the infection evaluated. The claw was sprayed with the formulation.

On day 3, the claw was cleaned and the infection evaluated. A second treatment was applied by spraying the formulation.

On day 10 the claw was cleaned and the infection evaluated. A significant reduction of the inflamed area was observed. No third treatment was necessary.

### C. Treatment of udder cleft dermatitis (UCD) - Field trial with qualitative evaluation

Field trials were conducted wherein cows diagnosed with UCD were treated using the composition of Example A described above. Five cows were treated in total, in two different farms.

In the first farm, four cows were treated during two weeks by spraying the composition on the wounds twice on the first day of treatment, and once per day for the remainder of the treatment. At the end of the treatment, the wounds had cured completely for three of the cows, whereas the wounds had cured for 80% for the other cow.

Similar treatment using a chlortetracycline antibiotic spray, Naxcel® (based on ceftiofur sodium); and Tylan® (tylosin) had no effect; or had only minor effects, wherein the symptoms recur after treatment.

In the second farm, one cow with very severe wounds was treated as follows:
- Days 0-2:: Once per day, the cow was positioned in a hoof trimming chute and treated by spraying the composition on the wounds. The skin was pulled open during spraying to obtain an optimal coverage within the skinfolds.
- Days 3-10: Once per day, the cow was positioned in a parallel parlor and treated by spraying the composition on the wounds. The skin was pulled open during spraying to obtain an optimal coverage within the skinfolds.
- Day 15:: The cow was positioned in the hoof trimming chute for inspection of the wounds.

Despite the severity, the wounds were cured completely after the above treatment.

No adverse effects were observed during or after treatment for any of the cows. The treatment does not require a withdrawal time and does not interfere with milk quality. Accordingly, the above treatment of UCD can be done in the milking parlor.

## Claims

1. A composition comprising copper and zinc chelates for use in the treatment or prevention of bacterial infections of epithelial cells in an animal and conditions related to bacterial infections of epithelial cells in an animal wherein said copper and zinc chelates are micronized and suspended in a liquid comprising less than 5 % (w/v) water, such that:
- the total amount of copper and zinc chelate in said formulation ranges from 5 to 50 percent by weight; and
- less than 5% (w/v) of said copper and zinc chelates are dissolved in said liquid.

2. The composition for use according to claim 1 wherein said infections are infections of any epithelial cell, of the skin and the hooves of animals, selected from the list consisting of: greasy pig disease (exudative epidermitis), porcine cutaneous spirochetosis, porcine necrotic ear syndrome, hairy hoof warts or Mortellaro's Disease (digital dermatitis), hoof rot (interdigital phlegmon), stable hoof rot (interdigital dermatitis), udder cleft dermatitis and M1 (early stage) and/or M2 (ulcerative stage) lesions on the skin or in the hoof and conditions selected from irritation (including reddening, swelling and other phenomena), pain and inflammation.

3. The composition for the use according to claim 1 or 2, wherein said copper and zinc chelates are dialkalimetal salts of copper(EDTA) and zinc(EDTA).

4. The composition for the use according to claim 3, wherein said dialkalimetal salts are the disodium salts of copper(EDTA) and zinc(EDTA).

5. The composition for the use according to any one of claims 1 to 4, wherein said micronized copper and zinc chelates are suspended in a non-aqueous liquid.

6. The composition for the use according to claim 5, wherein said liquid is selected from propanol, isopropanol, butanol, isobutanol, or a mixture thereof.

7. The composition for the use according to claim 6, wherein said liquid is propanol.

8. The composition for the use according to any one of claims 1 to 7, wherein the copper chelate : zinc chelate molar ratio of said copper and zinc chelates ranges from 2 : 1 to 1 : 2.

9. The composition for the use according to any one of claims 1 to 8, wherein said composition further comprises a silicate.

10. The composition for the use according to any one of claims 1 to 9, wherein less than 2% (w/v) of said copper and zinc chelates are dissolved in said liquid.

11. The composition for the use according to any one of claims 1 to 10, wherein said copper and zinc chelates are provided as microparticles, having a mean surface weighted diameter ranging from 0.5µm to 10µm.

12. The composition for the use according to any one of claims 1 to 11, for the treatment or prevention of an infection selected from exudative epidermitis, digital dermatitis, hoof rot, interdigital dermatitis, and udder cleft dermatitis.

13. A sprayable composition comprising micronized copper and zinc chelates suspended in a liquid comprising less than 5 % (w/v) water, wherein
- the total amount of copper and zinc chelate in said formulation ranges from 5 to 50 percent by weight; and
- less than 5% (w/v) of said copper and zinc chelates are dissolved in said liquid.

14. The sprayable composition according to claim 13, wherein said copper and zinc chelates are suspended in a skin-tolerable non-aqueous liquid.

15. The sprayable composition according to claim 13 or 14, wherein said liquid is selected from propanol, isopropanol, butanol, isobutanol, or a mixture thereof.

16. A spray can comprising a gas propellant and a composition according to any one of claims 1 to 15.

## Patentansprüche

1. Zusammensetzung, umfassend Kupfer- und Zinkchelate, zur Verwendung in der Behandlung oder Vorbeugung von bakteriellen Infektionen von Epithelzellen in einem Tier und Leiden, die mit bakteriellen Infektionen von Epithelzellen in einem Tier im Zusammenhang stehen,
wobei die Kupfer- und Zinkchelate mikronisiert und in einer Flüssigkeit, die weniger als 5% (w/v) Wasser umfasst, suspendiert sind, so dass:
- die Gesamtmenge an Kupfer- und Zinkchelaten in der Formulierung im Bereich von 5 bis 50 Gew.-% liegt; und
- weniger als 5% (w/v) der Kupfer- und Zinkchelate in der Flüssigkeit gelöst sind.

2. Zusammensetzung zur Verwendung nach Anspruch 1,
wobei es sich bei den Infektionen um Infektionen von einer beliebigen Epithelzelle, der Haut und den Hufen von Tieren handelt, ausgewählt aus der Aufreihung, die aus dem Folgenden besteht: nässende Oberhautentzündung bei Schweinen (exudative Epidermitis), Erdbeerkrankheit oder Stall-Huffäule (Dermatitis digitalis), Huffäule (Interdigitalphlegmone), Interdigitaldermatitis, "Udder Cleft Dermatitis" und Ml-Läsionen (Frühstadium) und/oder M2-Läsionen (ulzerierendes Stadium) an der Haut oder im Huf sowie Leiden, ausgewählt aus Reizung (einschließlich Rötung, Schwellen oder sonstigen Erscheinungen), Schmerzen und Entzündung.

3. Zusammensetzung zur Verwendung nach Anspruch 1 oder 2, wobei es sich bei den Kupfer- und Zinkchelaten um Dialkalimetallsalze von Kupfer-EDTA und Zink-EDTA handelt.

4. Zusammensetzung zur Verwendung nach Anspruch 3,
wobei es sich bei den Dialkalimetallsalzen um die Dinatriumsalze von Kupfer-EDTA und Zink-EDTA handelt.

5. Zusammensetzung zur Verwendung nach einem der Ansprüche 1 bis 4, wobei die mikronisierten Kupfer- und Zinkchelate in einer nichtwässrigen Flüssigkeit suspendiert sind.

6. Zusammensetzung zur Verwendung nach Anspruch 5,
wobei die Flüssigkeit aus Propanol, Isopropanol, Butanol, Isobutanol oder einer Mischung davon ausgewählt ist.

7. Zusammensetzung zur Verwendung nach Anspruch 6,
wobei es sich bei der Flüssigkeit um Propanol handelt.

8. Zusammensetzung zur Verwendung nach einem der Ansprüche 1 bis 7, wobei das Kupferchelat:Zinkchelat-Molverhältnis der Kupfer- und Zinkchelate im Bereich von 2:1 bis 1:2 liegt.

9. Zusammensetzung zur Verwendung nach einem der Ansprüche 1 bis 8, wobei die Zusammensetzung weiterhin ein Silikat umfasst.

10. Zusammensetzung zur Verwendung nach einem der Ansprüche 1 bis 9, wobei weniger als 2% (w/v) der Kupfer- und Zinkchelate in der Flüssigkeit gelöst sind.

11. Zusammensetzung zur Verwendung nach einem der Ansprüche 1 bis 10, wobei die Kupfer- und Zinkchelate als Mikropartikel mit einem mittleren oberflächengewichteten Durchmesser im Bereich von 0,5 µm bis 10 µm bereitgestellt werden.

12. Zusammensetzung zur Verwendung nach einem der Ansprüche 1 bis 11 für die Behandlung oder Vorbeugung einer Infektion, ausgewählt aus exudativer Epidermitis, Dermatitis digitalis, Huffäule, Interdigitaldermatitis und "Udder Cleft Dermatitis" ausgewählt ist.

13. Sprühbare Zusammensetzung, die in einer Flüssigkeit, die weniger als 5% (w/v) Wasser umfasst, suspendierte mikronisierte Kupfer- und Zinkchelate umfasst, wobei
- die Gesamtmenge an Kupfer- und Zinkchelaten in der Formulierung im Bereich von 5 bis 50 Gew.-% liegt; und
- weniger als 5% (w/v) der Kupfer- und Zinkchelate in der Flüssigkeit gelöst sind.

14. Sprühbare Zusammensetzung nach Anspruch 13, wobei die Kupfer- und Zinkchelate in einer hautverträglichen nichtwässrigen Flüssigkeit suspendiert sind.

15. Sprühbare Zusammensetzung nach Anspruch 13 oder 14, wobei die Flüssigkeit aus Propanol, Isopropanol, Butanol, Isobutanol oder einer Mischung davon ausgewählt ist.

16. Spraydose, die ein Treibgas und eine Zusammensetzung nach einem der Ansprüche 1 bis 15 umfasst.

## Revendications

1. Composition comprenant des chélates de cuivre et de zinc pour une utilisation dans le traitement ou la prévention d'infections bactériennes de cellules épithéliales chez un animal et de conditions liées à des infections bactériennes de cellules épithéliales chez un animal,
dans laquelle lesdits chélates de cuivre et de zinc sont micronisés et mis en suspension dans un liquide comprenant moins de 5% (p/v) d'eau, de sorte que
- la quantité totale de chélates de cuivre et de zinc dans ladite formulation aille de 5 à 50% en poids ; et
- moins de 5% (p/v) desdits chélates de cuivre et de zinc soient solubilisés dans ledit liquide.

2. Composition pour une utilisation selon la revendication 1, dans laquelle lesdites infections sont des infections d'une cellule épithéliale quelconque, de la peau et des sabots des animaux, choisies parmi la liste constituée par : le syndrome du porc gras (épidermite exsudative), la spirochétose cutanée du porc, le syndrome de la nécrose d'oreilles du porc, les verrues velues du sabot ou la maladie de Mortellaro (dermatite digitée), la pourriture du sabot (phlegmon interdigité), la pourriture stable du sabot (dermatite interdigitée), la dermatite au sillon de la mamelle et les lésions M1 (stade précoce) et/ou M2 (stade ulcératif) sur la peau ou dans le sabot et les conditions choisies parmi les irritations (y compris les rougissements, les tuméfactions et autres phénomènes), les douleurs et les inflammations.

3. Composition pour l'utilisation selon la revendication 1 ou 2, dans laquelle lesdits chélates de cuivre et de zinc sont des sels de di-métaux alcalins de cuivre (EDTA) et de zinc (EDTA).

4. Composition pour l'utilisation selon la revendication 3, dans laquelle lesdits sels de di-métaux alcalins sont les sels disodiques de cuivre (EDTA) et de zinc (EDTA).

5. Composition pour l'utilisation selon l'une quelconque des revendications 1 à 4, dans laquelle lesdits chélates de cuivre et de zinc micronisés sont mis en suspension dans un liquide non aqueux.

6. Composition pour l'utilisation selon la revendication 5, dans laquelle ledit liquide est choisi parmi le propanol, l'isopropanol, le butanol, l'isobutanol, ou un mélange de ceux-ci.

7. Composition pour l'utilisation selon la revendication 6, dans laquelle ledit liquide est le propanol.

8. Composition pour l'utilisation selon l'une quelconque des revendications 1 à 7, dans laquelle le rapport molaire chélate de cuivre:chélate de zinc desdits chélates de cuivre et de zinc va de 2:1 à 1:2.

9. Composition pour l'utilisation selon l'une quelconque des revendications 1 à 8, dans laquelle ladite composition comprend en outre un silicate.

10. Composition pour l'utilisation selon l'une quelconque des revendications 1 à 9, dans laquelle moins de 2% (p/v) desdits chélates de cuivre et de zinc sont solubilisés dans ledit liquide.

11. Composition pour l'utilisation selon l'une quelconque des revendications 1 à 10, dans laquelle lesdits chélates de cuivre et de zinc sont fournis sous forme de microparticules, ayant un diamètre moyen pondéré en surface allant de 0,5 µm à 10 µm.

12. Composition pour l'utilisation selon l'une quelconque des revendications 1 à 11, pour le traitement ou la prévention d'une infection choisie parmi l'épidermite exsudative, la dermatite digitée, la pourriture du sabot, la dermatite interdigitée, et la dermatite au sillon de la mamelle.

13. Composition pulvérisable comprenant des chélates de cuivre et de zinc micronisés et mis en suspension dans un liquide comprenant moins de 5% (p/v) d'eau, dans laquelle
- la quantité totale de chélates de cuivre et de zinc dans ladite formulation va de 5 à 50% en poids ; et
- moins de 5% (p/v) desdits chélates de cuivre et de zinc sont solubilisés dans ledit liquide.

14. Composition pulvérisable selon la revendication 13, dans laquelle lesdits chélates de cuivre et de zinc sont mis en suspension dans un liquide non aqueux et tolérable par la peau.

15. Composition pulvérisable selon la revendication 13 ou 14, dans laquelle ledit liquide est choisi parmi le propanol, l'isopropanol, le butanol, l'isobutanol, ou un mélange de ceux-ci.

16. Atomiseur comprenant un gaz propulseur et une composition selon l'une quelconque des revendications 1 à 15.
